(19) Europäisches Patentamt
European Patent Office
Office européen des brevets

(11) Veröffentlichungsnummer: **0 023 022**
**B1**

(12) # EUROPÄISCHE PATENTSCHRIFT

(45) Veröffentlichungstag der Patentschrift: **11.04.84**

(51) Int. Cl.³: **C 09 B 57/02**, C 07 D417/04, C 07 D405/04, C 07 D277/64, C 07 D235/16

(21) Anmeldenummer: **80104119.5**

(22) Anmeldetag: **15.07.80**

(54) **Cumarinverbindungen, Verfahren zu deren Herstellung sowie die dafür verwendeten Zwischenprodukte an sich und ihre Verwendung zum Färben oder Bedrucken von Textilien.**

(30) Priorität: **20.07.79 CH 6772/79**

(43) Veröffentlichungstag der Anmeldung: **28.01.81 Patentblatt 81/04**

(45) Bekanntmachung des Hinweises auf die Patenterteilung: **11.04.84 Patentblatt 84/15**

(84) Benannte Vertragsstaaten: **CH DE FR GB LI**

(56) Entgegenhaltungen:
**DE-A- 2 030 507**
**DE-A- 2 058 877**
**DE-A- 2 065 076**
**DE-A- 2 364 478**
**FR-A- 1 511 523**
**FR-A- 2 343 786**
**Die Akte enthält technische Angaben, die nach dem Eingang der Anmeldung eingereicht wurden und die nicht in dieser Patentschrift enthalten sind.**

(73) Patentinhaber: **CIBA-GEIGY AG**
**Postfach**
**CH-4002 Basel (CH)**

(72) Erfinder: **Schwander, Hansrudolf, Dr.**
**Unterm Schellenberg 189 CH-4125 Riehen CH (CH)**
Erfinder: **Zickendraht, Christian, Dr.**
**Enzianstrasse 2**
**CH-4102 Binningen (CH)**

(74) Vertreter: **Berg, Wilhelm, Dr. et al**
**Mauerkircher Strasse 45**
**D-8000 München 80 (DE)**

**EP 0 023 022 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

Cumarinverbindungen, Verfahren zu deren Herstellung sowie die dafür verwendeten Zwischenprodukte an sich und ihre Verwendung zum Färben oder Bedrucken von Textilien

Gegenstand der Erfindung sind neue Cumarinverbindungen der Formel I

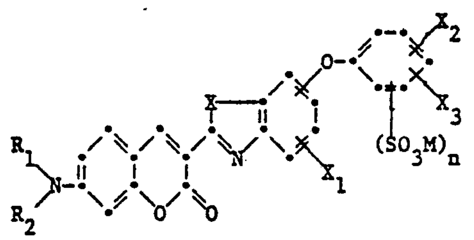

(I)

worin bedeuten :

$R_1$ und $R_2$ unabhängig voneinander einen Alkylrest mit 1 bis 4 Kohlenstoffatomen, X das —S-Atom oder die —NH-Gruppe, $X_1$ Wasserstoff oder Halogen, vorzugsweise Wasserstoff, $X_2$ und $X_3$ unabhängig voneinander Wasserstoff, einen Alkylrest mit 1 bis 4 Kohlenstoffatomen oder Halogen, vorzugsweise je Wasserstoff, n entweder 0 oder 1, und M Wasserstoff oder ein Aequivalent eines Kations.

Bedeuten $R_1$, $R_2$, $X_2$ und/oder $X_3$ Alkylreste mit 1 bis 4 Kohlenstoffatomen, so können diese verzweigt oder unverzweigt sein. Es handelt sich z. B. um den Methyl-, Aethyl, n- und iso-Propyl- und n-, sec- oder tert. Butylrest. Die Alkylreste in der Stellung von $R_1$ und $R_2$ sind vorzugsweise identisch und bedeuten insbesondere je den Aethylrest.

Bedeuten $X_1$, $X_2$ und/oder $X_3$ Halogen, so handelt es sich um Brom-, Fluor- und vorzugsweise Chlor.

M steht für Wasserstoff oder ein beliebiges bei anionischen Farbstoffen übliches Kation, z. B. für Alkalimetall, Ammonium, Mono-, Di- und Tri-($C_2$-$C_3$-alkanol)-ammonium oder Mono-, Di-, Tri- oder Tetra-($C_1$-$C_4$-alkyl)-ammonium, vorzugsweise jedoch für Alkalimetall (Lithium, Natrium, Kalium).

In bevorzugten Cumarinverbindungen bedeuten n die Zahl 1, $R_1$ und $R_2$ je Aethyl und $X_1$, $X_2$ und $X_3$ je Wasserstoff.

Die erfindungsgemässen Cumarinverbindungen der Formel I werden in an sich bekannter Weise hergestellt, z. B. a) in dem man heterocyclische Verbindungen der Formel II

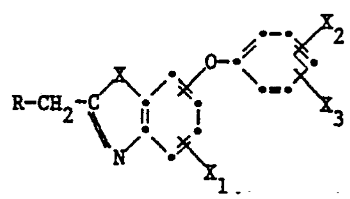

(II)

worin X, $X_1$, $X_2$ und $X_3$ die angegebene Bedeutung haben und R Alkoxycarbonyl, Carbamoyl oder CN bedeutet mit einem Aldehyd der Formel III

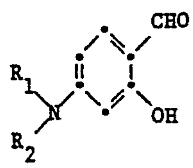

(III)

worin $R_1$ und $R_2$ die angegebene Bedeutung haben, kondensiert und das Kondensationsprodukt gegebenenfalls sulfiert, oder b) indem man eine heterocyclische Verbindung der Formel II bei Raumtemperatur (ca. 15-25 °C) sulfiert, z. B. mit Schwefelsäuremonohydrat und nach der Sulfierung mit einem Aldehyd der Formel III kondensiert ; diese Variante kommt somit nur bei Cumarinverbindungen der Formel I in Betracht, worin n die Zahl 1 bedeutet.

Bei der Kondensationsreaktion a) arbeitet man vorteilhaft in Gegenwart eines organischen Lösungsmittels und in Gegenwart einer starken Säure.

Die Kondensation erfolgt bei Temperaturen von 0° bis etwa 160 °C, vorzugsweise bei 20 bis 70 °C und ist u. a. abhängig vom Siedepunkt des eingesetzten Lösungsmittels. In der Regel wird man die Temperatur so niedrig wie möglich wählen.

Die heterocyclischen Verbindungen der Formel II sind neu. Man erhält z. B. derartige Produkte worin X die —NH-Gruppe darstellt, wenn man ein Diamin der Formel

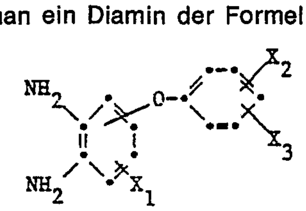

mit einem Iminoäther der Formel

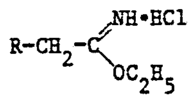

worin $X_1$, $X_2$, $X_3$ und R die angegebene Bedeutung haben umsetzt ; bedeutet X in der Formel II das —S-Atom so erhält man diese Verbindungen beispielsweise indem man einem o-Amino-mercapto-diphenyläther der Formel

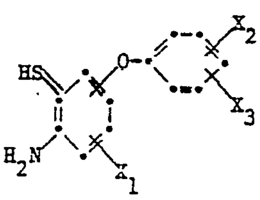

mit z. B. Malonsäuredinitril umsetzt.

Beispiele für derartige heterocyclische Produkte der Formel II sind :

5-Chlor-6-phenoxy-benzimidazol-2-cyanmethyl,
6-Phenoxy-benzimidazol-2-äthoxycarbonylmethyl,
6-(2'-Methyl)-phenoxy-benzimidazol-2-äthoxycarbonylmethyl,
6-(4'-Methyl)-phenoxy-benzimidazol-2-äthoxycarbonylmethyl,
6-(2',4'-Dimethyl)-phenoxy-benzimidazol-2-äthoxycarbonylmethyl,
6-(2'-Chlor)-phenoxy-benzimidazol-2-äthoxycarbonylmethyl,
6-(4'-Chlor)-phenoxy-benzimidazol-2-äthoxycarbonylmethyl,
6-(2',4'-Dichlor)-phenoxy-benzimidazol-2-äthoxycarbonylmethyl,
6-Phenoxy-benzimidazol-2-cyanmethyl,
6-(2'-Methyl)-phenoxy-benzthiazol-2-cyanmethyl,
6-(4'-Methyl)-phenoxy-benzthiazol-2-cyanmethyl,
6-(2',4'-Dimethyl)-phenoxy-benzthiazol-2-cyanmethyl,
6-(2'-Chlor)-phenoxy-benzthiazol-2-cyanmethyl,
6-(4'-Chlor)-phenoxy-benzthiazol-2-cyanmethyl,
6-(2',4'-Dichlor)-phenoxy-benzthiazol-2-cyanmethyl,
4-Phenoxy-7-chlor-benzthiazol-2-cyanmethyl,
4-(2'-Methyl)-phenoxy-7-chlor-benzthiazol-2-cyanmethyl,
4-(2',4'-Dimethyl)-phenoxy-7-chlor-benzthiazol-2-cyanmethyl, und
6-Phenoxy-benzthiazol-2-cyanmethyl.

Die Aldehyde der Formel III sind bekannt und nach bekannten Methoden herstellbar. Besonders bevorzugt wird im erfindungsgemässen Verfahren N-Diäthylaminosalicylaldehyd.

Als Lösungsmittel kommen gegen Säuren stabile Lösungsmittel in Frage. Einerseits kann man mit Wasser mischbare Lösungsmittel wie z. B. Alkohole (Methanol, Aethanol, Isopropylalkohol), Dioxan, Methoxyäthanol, Aethoxyäthanol, 1,2-Dimethoxyäthan, Dimethylformamid, Tetramethylharnstoff, Sulfolan und Dimethylsulfoxid verwenden. Auch Gemische der genannten Lösungsmittel können verwendet werden. Andererseits kann man auch mit Wasser nicht-mischbare Lösungsmittel verwenden, wie z. B. aromatische Kohlenwasserstoffe wie Benzol, Toluol oder Xylol, aliphatische und aromatische Chlorkohlenwasserstoffe wie Tetrachlorkohlenstoff, Trichloräthylen, Aethylenchlorid, Methylenchlorid, Chloroform, Trichloräthan, Tetrachloräthan, Perchloräthylen, Mono- oder Dichlorbenzol.

Als Säuren kommen vor allem in Frage : Mineralsäuren wie Halogenwasserstoffsäuren, Schwefelsäure und Phosphorsäure aber auch starke organische Säuren wie Ameisensäure, Essigsäure oder Chloressigsäure. Bevorzugt wird Eisessig verwendet.

Die erhaltenen Kondensationsprodukte aus II und III d. h. die Cumarinverbindungen der Formel I, worin n 0 bedeutet, eignen sich zur Verwendung als Dispersionsfarbstoffe ; sie können noch zu Cumarinverbindungen der Formel I sulfoniert werden, worin n die Zahl 1 bedeutet, welche sich zur Verwendung als saure Farbstoffe eignen.

Die Sulfonierung der Kondensationsprodukte erfolgt dabei nach bekannter Art und Weise z. B. mit $SO_3$ oder einem $SO_3$-abgebenden Mittel oder in Schwefelsäure. Die Behandlung mit $SO_3$ oder mit Chlorsulfonsäure kann z. B. in einem inerten Lösungsmittel wie Chloroform, 1,2-Dichloräthan, Nitrobenzol bei Temperaturen von 0° bis 70 °C vorzugsweise bei 15-30 °C ausgeführt werden. Ueblicherweise arbeitet man jedoch mit konzentrierter, etwa 96 bis 100 %iger Schwefelsäure oder mit Oleum, das vorteilhaft einen 30-75 %igen $SO_3$-Gehalt aufweist bei den genannten Temperaturen.

Die erhaltenen Cumarin-Verbindungen der Formel I können auf übliche Weise aufgearbeitet werden

wobei z. B. eine freie Säure gegebenenfalls während dem Aufarbeiten z. B. nach Giessen des Sulfonierungsproduktes auf Eissole zum entsprechenden Salz neutralisiert werden kann.

Die neuen Cumarinverbindungen der Formel I sind als disperse Farbstoffe (n = 0) oder als anionische Farbstoffe (n = 1) zum Färben und Bedrucken, vor allem im Bekleidungssektor verwendbar.

Werden die Cumarinverbindungen der Formel I als Dispersionsfarbstoffe (n = 0) verwendet, so eignen sie sich für das Färben und Bedrucken von mit Dispersionsfarbstoffen färbbaren Materialien, insbesondere Textilmaterialien, vor allem von synthetischem und halbsynthetischem Textilmaterial z. B. aus Cellulose-2 1/2-acetat, Cellulosetriacetat, Polyestern und Polyamid.

Die Färbung der genannten Fasermaterialien erfolgt mit den erfindungsgemässen Dispersionsfarbstoffen aus wässriger Dispersion. Es ist deshalb zweckmässig, die als Dispersionsfarbstoffe verwendbaren Vertreter durch Vermahlen mit Textilhilfsmitteln, wie z. B. Dispergiermittel, und möglicherweise Mahlhilfsstoffen fein zu zerteilen. Durch anschliessende Trocknung erhält man aus dem Textilhilfsmittel und dem Farbstoff eine Farbstoffzubereitung.

Beispielsweise sind als Dispergiermittel genannt : nicht-ionische Dispergiermittel, wie Anlagerungsprodukte von 8 Mol Aethylenoxyd an 1 Mol p-tert. Octylphenol, von 15 bzw. 6 Mol Aethylenoxyd an Rizinusöl, von 20 Mol Aethylenoxyd an den Alkohol $C_{16}H_{33}OH$, Aethylenoxyd-Anlagerungsprodukte an Di[α-phenyläthyl]-phenole, Polyäthylenoxyd-tert. dodecyl-thioäther, oder Anlagerungsprodukte von 15 bzw. 30 Mol Aethylenoxyd an 1 Mol $C_{12}H_{25}NH_2$ oder $C_{18}H_{37}NH_2$ ; dann anionische Dispergiermittel, wie z. B. Schwefelsäureester von Alkoholen der Fettreihe mit 8 bis 20 Kohlenstoffatomen, von Aethylenoxydaddukten von entsprechenden Fettsäureamiden, oder von alkylierten Phenolen mit 8 bis 12 Kohlenstoffatomen im Alkylrest ; Sulfonsäureester mit Alkylresten mit 8 bis 20 Kohlenstoffatomen ; Sulfatierungsprodukte von ungesättigten Fetten und Oelen ; Phosphorsäureester mit Alkylresten mit 8 bis 20 Kohlenstoffatomen ; Fettsäureseifen, ferner Alkylarylsulfonate, Kondensationsprodukte des Formaldehyds mit Naphthalinsulfonsäure und Ligninsulfonate ; ferner kommen noch kationische Dispergiermittel in Frage, wie z. B. quaternäre Ammoniumverbindungen, welche Alkyl- oder Aralkylreste mit 8 bis 20 Kohlenstoffatomen enthalten.

Die Farbstoffpräparate können zusätzlich zu den Dispergiermitteln noch organische Lösungsmittel, insbesondere über 100 °C siedende Lösungsmittel, welche vorzugsweise mit Wasser mischbar sind, wie Mono- und Dialkylglykoläther, Dioxan, Dimethylformamid oder -acetamid, Tetramethylensulfon oder Dimethylsulfoxyd, enthalten. Man kann vorteilhaft Farbstoff, Dispergiermittel und Lösungsmittel miteinander vermahlen.

Die neuen Cumarinverbindungen eignen sich ferner als Farbstoffe für den Druck auf Polyestermaterialien z. B. nach dem Hochtemperaturdampfverfahren.

Die Färbung der Polyesterfasern mit den erfindungsgemässen Dispersionsfarbstoffen erfolgt nach den für Polyestermaterialien üblichen Verfahren. Polyester aromatischer Polycarbonsäure mit mehrwertigen Alkoholen färbt man vorzugsweise bei Temperaturen von über 100 °C unter Druck. Die Färbung kann aber auch beim Siedepunkt des Färbebades in Gegenwart von Farbüberträgern, beispielweise Phenylphenole, Polychlorbenzolverbindungen oder ähnlichen Hilfsmitteln, durchgeführt oder nach dem Thermosolverfahren, d. h. Foulardieren mit anschliessender Nachbehandlung in der Hitze, z. B. Thermofixierung, bei 180 bis 210 °C, vorgenommen werden. Cellulose-2 1/2-acetatfasern färbt man vorzugsweise bei Temperaturen von 80 bis 85 °C, während Cellulosetriacetatfasern mit Vorteil beim Siedepunkt des Färbebades gefärbt werden. Beim Färben von Cellulose-2 1/2-acetat erübrigt sich die Verwendung von Farbüberträgern.

Man erhält dabei Ausfärbungen die gekennzeichnet sind durch gute Allgemeinechtheiten, wie vor allem durch eine gute Sublimationsechtheit, durch einen guten Farbstoffaufbau, eine gute pH-Stabilität, gute Lichtechtheit und sehr grosser Farbstärke ; die Ausfärbungen weisen vor allem eine sehr gute Fluoreszenz auf.

Die erfindungsgemässen Cumarinverbindungen der Formel I, worin n = 1 bedeutet haben anionischen Charakter und sind als anionische Farbstoffe verwendbar ; sie eignen sich für das Färben und Bedrucken von mit anionischen Farbstoffen färbbaren Substraten, vor allem Polyamidmaterialien und Wolle, ferner sind sie geeignet als Laserfarbstoffe und durch Beismischen zu Pigmenten zur Herstellung von Fluoreszenzpigmenten.

Das Färben kann nach beliebigen für das entsprechende Substrat geeigneten, kontinuierlichen oder diskontinuierlichen Methoden (z. B. Auszieh-, Klotz- oder Druckverfahren) erfolgen. Mit den erfindungsgemässen sauren Cumarinfarbstoffen werden Ausfärbungen erhalten, die gute Allgemeinechtheiten aufweisen wie vor allem sehr gute Egalität, guter Farbstoffaufbau, gute Nassechtheiten, grosse Farbstärke, gute Lichtechtheit, gutes Migriervermögen und vor allem eine sehr gute Fluoreszenz ; darüber hinaus sind die Färbungen für Kombinationsfärbungen geeignet und ergeben zudem streifenfreie Ausfärbungen.

All diese Textilmaterialien können in den verschiedensten Verarbeitungsformen wie Gewirke, Gewebe, Garne und Fasern sowie fertigen Artikeln wie Hemden vorliegen.

Die sauren Cumarinfarbstoffe der Formel I eignen sich auch z. B. in Form ihrer Salze, beispielsweise Alkalimetallsalze, wie Natrium- oder Kaliumsalze zum Spinnfärben von in organischen vorzugsweise polaren Lösungsmitteln gelösten Kunststoffmassen und zum Färben von Kunststoffen und Lacken, z. B. Nitro- oder Vinyllacken.

Aus der FR-A-2 343 786 sind disperse Cumarinfarbstoffe ähnlicher Struktur, jedoch ohne eine Phenoxysubstitution bekannt und in der DE-A-2 065 076 sind analoge saure und disperse Cumarinfarbstoffe, jedoch mit einem Oxazolrest anstelle eines Benzimidazol- bzw. Benzthiazolrestes beschrieben. Es war überraschend, dass die erfindungsgemässen Phenoxysubstituierten Cumarinbenzimidazol- bzw. Cumarinbenzthiazolfarbstoffe gegenüber diesen nächstvergleichbaren und vorbekannten sauren Farbstoffen eine verbesserte Nassechtheit und ein besseres Aufziehvermögen und gegenüber diesen nächstvergleichbaren und vorbekannten dispersen Farbstoffen eine verbesserte Sublimationsechtheit aufweisen.

In den nachfolgenden Beispielen bedeuten die Teile, sofern nichts anderes angegeben wird, Gewichtsteile, die Prozente Gewichtsprozente und die Temperaturen sind in Celsiusgraden angegeben. Sie sollen die Erfindung erläutern ohne sie jedoch in irgendeiner Form zu beschränken.

Beispiel 1

Man rührt ein Gemisch aus 21,8 g der Verbindung der Formel

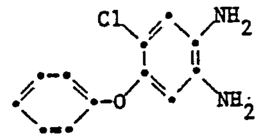

(erhalten durch katalytische Reduktion von 2-Nitro-4-chlor-5-phenoxyanilin) und 20,5 g des Imino-aethers der Formel

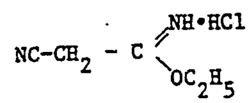

in 150 ml Dioxan während 12 Std. bei Raumtemperatur. Anschliessend entfernt man das Dioxan im Vakuum mittels eines Rotationsverdampfers und behandelt den Rückstand in einer Reibschale sechsmal mit je 50 ml sauer gestelltem Wasser, worauf man das Rohprodukt auf einer Nutsche abfiltriert, mit Wasser neutral wäscht und im Vakuum bei 70° trocknet.

Zur Reinigung wird das Rohprodukt aus 8 Teilen Essigester umkristallisiert.

Das so erhaltene Produkt der Formel

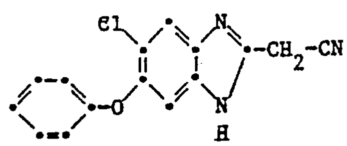

bildet farblose Kristalle, welche sich in Aethanol in der Wärme lösen.

Verwendet man, bei sonst gleichem Vorgehen, anstelle des oben angeführten Diamins eine aequivalente Menge der Verbindung der Formel

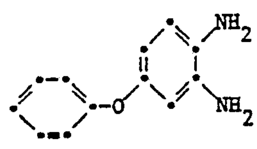

(erhalten durch Nitrierung von 4-Phenoxy-acetanilid, alkalische Hydrolyse der Nitroverbindung und anschliessende katalytische Reduktion) und anstelle des oben angeführten Iminoaethers eine aequivalente Menge des Iminoaethers der Formel

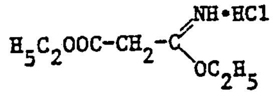

so erhält man die Verbindung der Formel

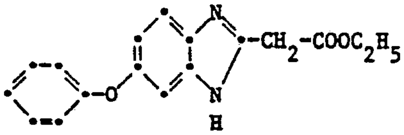

welche ähnliche Eigenschaften wie das oben aufgeführte Endprodukt aufweist.

Verwendet man bei ansonst gleichem Vorgehen die in der folgenden Tabelle I unter Kolonne I aufgeführten Diamine, so erhält man die unter Kolonne II aufgeführten Benzimidazol-Derivate.

Tabelle I

| Nr. | I | II |
|---|---|---|
| | Diamin | Benzimidazole |
| 2 | 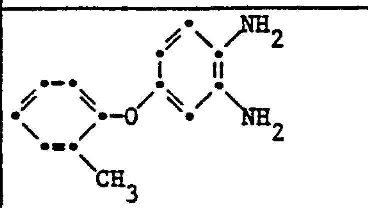 | 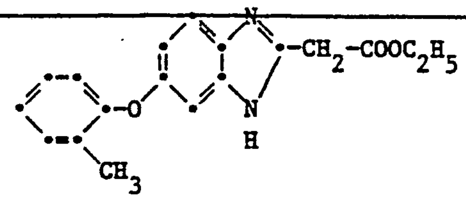 |
| 3 | 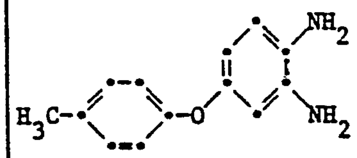 | 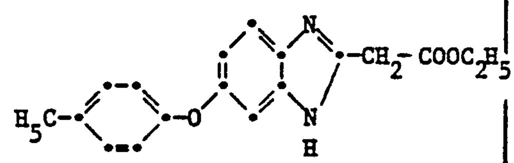 |
| 4 | 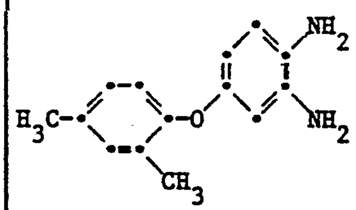 | 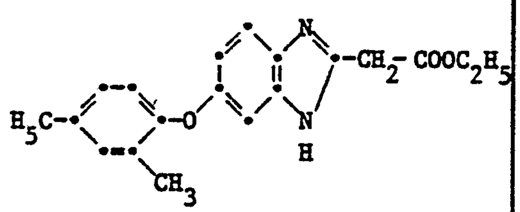 |
| 5 | 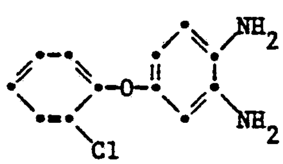 | 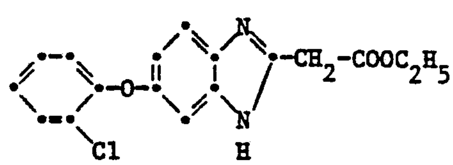 |
| 6 | 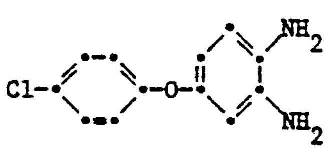 | 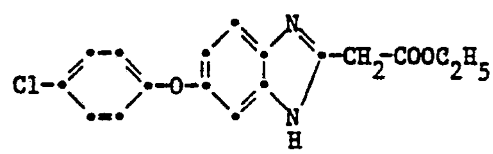 |
| 7 | 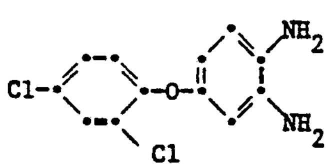 | 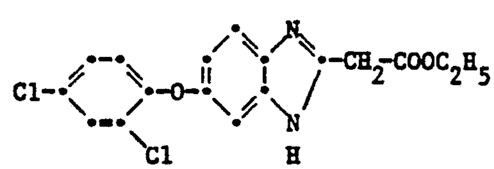 |

Beispiel 8

Ein Gemisch, bestehend aus 5,35 g der Verbindung der Formel

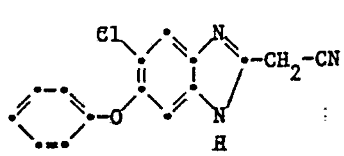

4,05 g N-Diäthylamino-salicylaldehyd, 30 ml Aethanol, 0,10 ml Piperidin und 0,10 ml Eisessig wird

während 6 Stunden unter Rückfluss gerührt. Man gibt hierauf 6 ml 36 %ige Salzsäure zu und rührt weitere 5 Stunden unter Rückfluss. Anschliessend lässt man auf Raumtemperatur abkühlen, filtriert das ausgefallene Produkt ab und wäscht dieses zuerst mit Aethanol bis das Filtrat eine hellgelbe Farbe zeigt, dann mit heissem Wasser. Nach Trocknung im Vakuum bei 70° erhält man die Cumarinverbindung der Formel

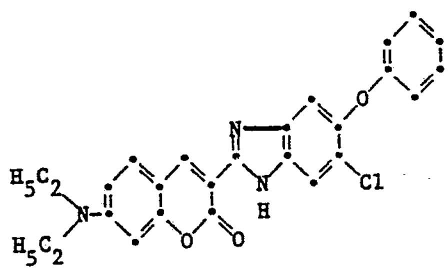

als braungelbes Pulver, welches sich in organischen Lösungsmitteln wie z. B. Chlorbenzol grünstichig-gelb mit intensiver Fluoreszenz löst.

Nach üblichen Methoden als Dispersionsfarbstoff auf Polyester appliziert erhält man grünstichig-gelbe fluoreszierende Färbungen, welche sich durch eine sehr gute Sublimationsechtheit und eine gute pH-Beständigkeit auszeichnen.

Beispiel 9

Man rührt ein Gemisch, bestehend aus 6 g der Verbindung der Formel

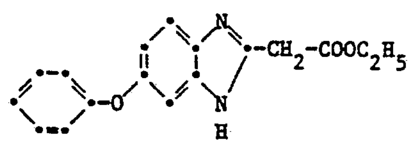

4,05 g N-Diäthylamino-salicylaldehyd, 30 ml Aethanol, 0,10 ml Piperidin und 0,10 ml Eisessig während 12 Std. unter Rückflusstemperatur. Anschliessend lässt man auf Raumtemperatur abkühlen, filtriert das ausgefallene Produkt ab und wäscht dieses erst mit Aethanol, bis das Filtrat eine hellgelbe Farbe zeigt, dann noch mit Wasser. Nach Trocknung im Vakuum bei 70° erhält man die Cumarinverbindung der Formel

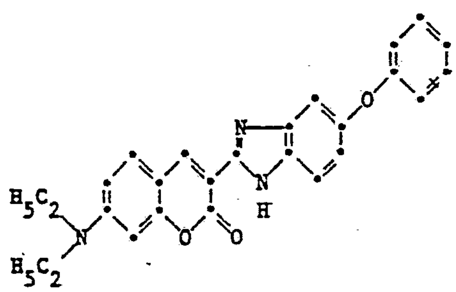

als orange-gelbes Pulver, welches sich im organischen Lösungsmittel z. B. Chlorbenzol mit grünstichiggelber Farbe und intensiver Fluoreszenz löst.

Die mit diesem Farbstoff auf Polyester nach den für Dispersionsfarbstoffe üblichen Färbemethoden erzeugten Färbungen sind sehr gut sublimationsecht und pH-beständig.

Verwendet man, bei ansonst gleichem Vorgehen, anstelle der oben aufgeführten Benzimidazol-Verbindung die Benzimidazol-Verbindungen der Tabelle I, Kolonne II, so erhält man die in Tabelle II aufgeführten Cumarinverbindungen, welche ähnliche Eigenschaften aufweisen.

(Siehe Tabelle II Seite 8 f.)

Tabelle II

| Nr. | Cumarinverbindung | Nuance auf Polyester |
|---|---|---|
| 10 | 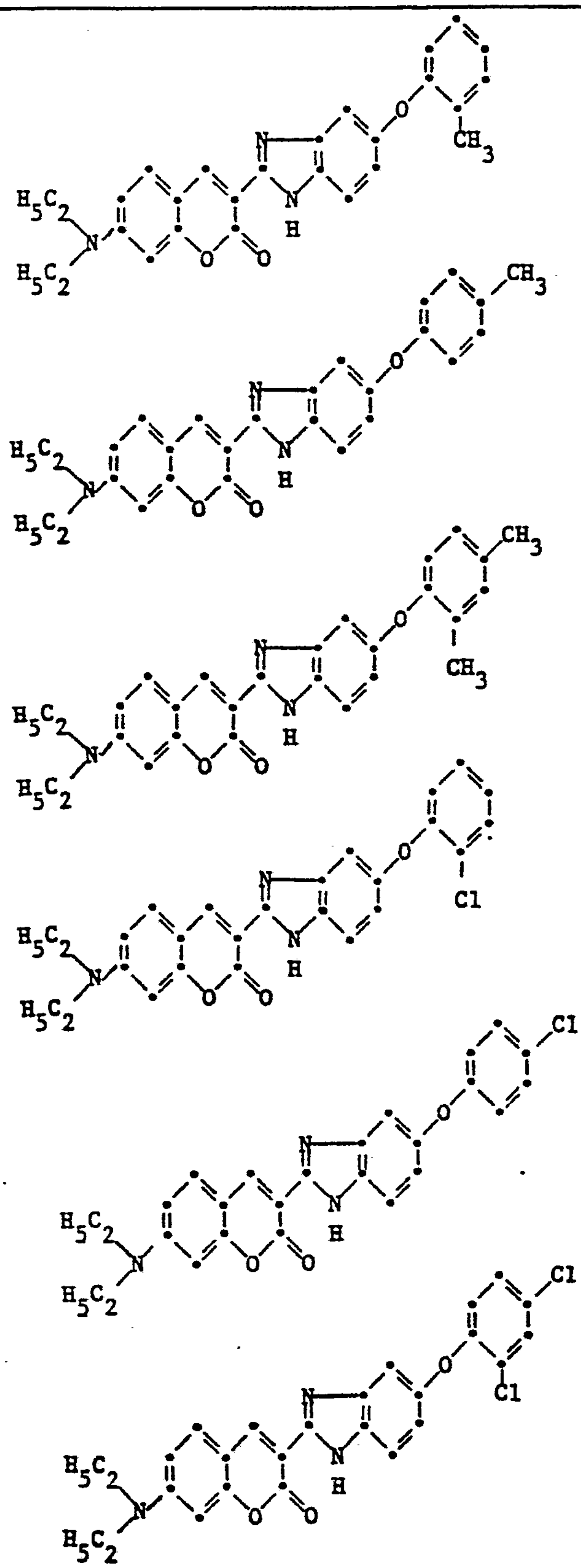 | grünstichig-gelb |
| 11 | | do. |
| 12 | | do. |
| 13 | | do. |
| 14 | | grünstichig-gelb |
| 15 | | do. |

Beispiel 16

49 g Phenoxy-phenylthioharnstoff (Am. Soc. *48* 1073) werden in 200 ml Chloroform suspendiert und innert einer Stunde eine Lösung von 40 g Brom in 100 ml Chloroform unter Rühren zugetropft. Hierauf rührt man das Ganze zwei Stunden am Rückfluss und filtriert dann das 5-Phenoxy-2-amino-benzthiazolhydrobromid ab. Durch Lösen in Wasser und Ausfällen mit Natriumhydroxyd-Lösung wird die freie Base der Formel

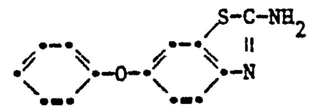

erhalten.

97 g 5-Phenoxy-2-amino-benzthiazol werden mit einer Lösung aus 100 g Kalium-hydroxyd in 600 ml Wasser in einem Nickel-Autoklaven zwei Stunden bei 160° gerührt. Dabei wird ein Maximaldruck von 5 atü gemessen. Der Autoklaveninhalt wird dann filtriert, das Filtrat mit gleichem Volumen Eis versetzt und mit Salzsäure neutralisiert. Der auskristallisierte 3-Merkapto-4-amino-diphenyläther wird abgesaugt und die Paste in 400 ml Methylalkohol suspendiert. Nach Zugabe von 35 g Malodinitril wird das Ganze 24 Stunden bei Raumtemperatur gerührt und das so erhaltene 5-Phenoxy-2-cyanmethyl-benzthiazol der Formel

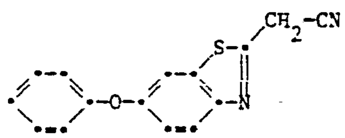

abgesaugt, gewaschen und getrocknet. Es bildet farblose feine Nadeln welche sich in Aethanol in der Wärme lösen.

Verwendet man anstelle von Phenoxy-phenylthioharnstoff bei ansonst gleichem Vorgehen die in Tabelle III unter Kolonne I aufgeführten Thioharnstoffe (darstellbar nach der Methode in Am. Soc. *48*, 1073) so erhält man die unter Kolonne II aufgeführten Benzthiazol-Verbindungen.

Tabelle III

| Nr. | I | II |
|---|---|---|
| | Thioharnstoffe | Benzthiazole |
| 17 | 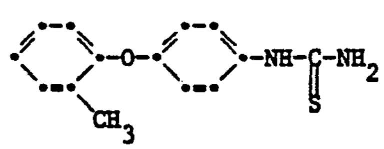 | 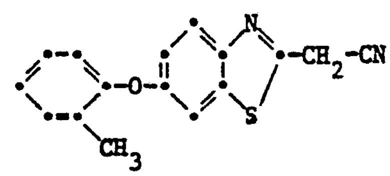 |
| 18 | 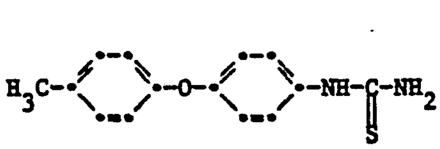 | 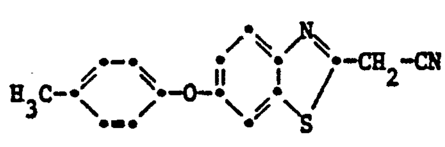 |

Tabelle III (Fortsetzung)

| Nr. | I<br>Thioharnstoffe | II<br>Benzthiazole |
|---|---|---|
| 19 | 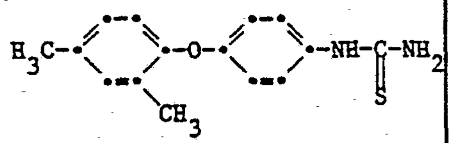 | 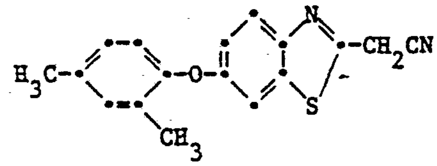 |
| 20 | 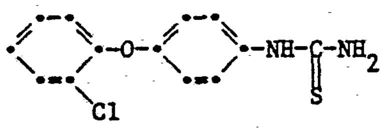 | 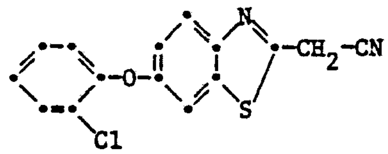 |
| 21 | 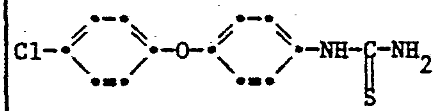 | 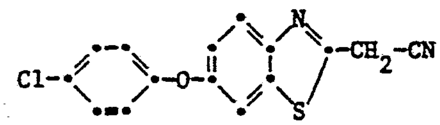 |
| 22 | 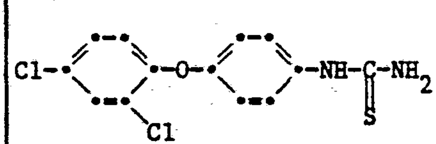 | 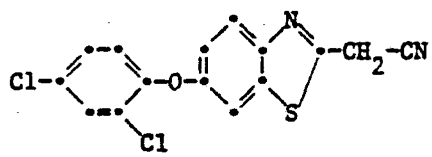 |
| 23 | 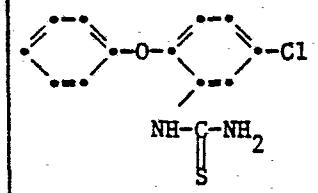 | 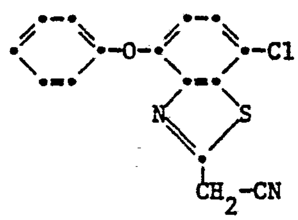 |
| 24 | 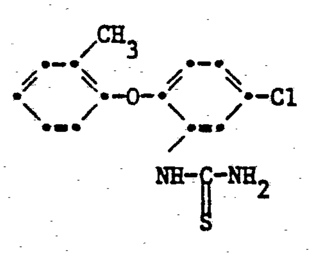 | 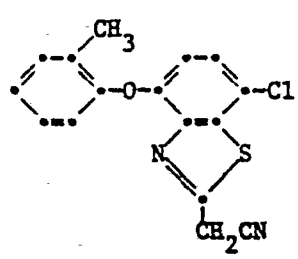 |
| 25 | 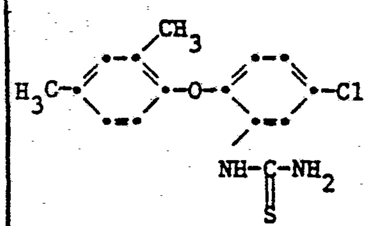 | 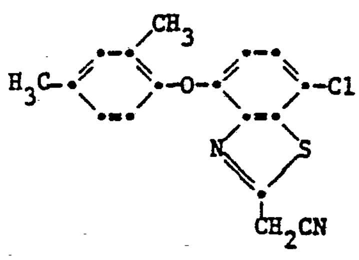 |

Beispiel 26

Man rührt ein Gemisch, bestehend aus 5,30 g der Verbindung der Formel

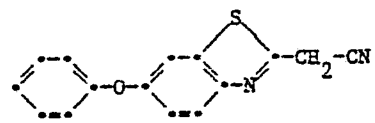

4,05 g N-Diäthylaminosalicylaldehyd, 30 ml Aethanol, 0,10 ml Piperidin und 0,10 ml Eisessig während 9 Stunden unter Rückflusstemperatur. Anschliessend gibt man 2 ml 36 %ige Salzsäure zu und rührt noch weitere 2 Stunden unter Rückfluss. Nach Erkalten auf Raumtemperatur filtriert man das ausgefallene Produkt ab und wäscht dieses mit Aethanol bis das Filtrat hell gelb abläuft und zuletzt noch mit Wasser. Nach Trocknung im Vakuum bei 70° erhält man die Cumarinverbindung der Formel

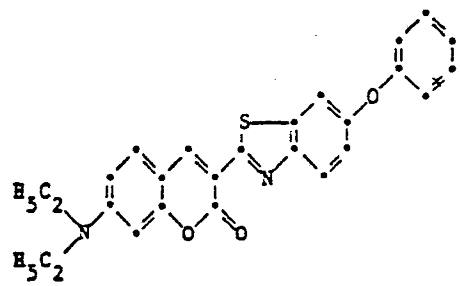

als organge-gelbes Pulver, welches sich in Chlorbenzol grünstichig-gelb mit intensiver Fluoreszenz löst.

Als Dispersionsfarbstoff auf Polyester, nach den üblichen Färbemethoden appliziert erhält man brillante grünstichig-gelbe Färbungen mit intensiver Fluoreszenz. Die Färbungen sind gut sublimations- und lichtecht und weisen eine gute pH-Beständigkeit auf.

Verwendet man, bei ansonst gleichem Vorgehen, anstelle der oben aufgeführten Benzthiazol-Verbindung aequivalente Mengen der Benzthiazol-Verbindungen der Tabelle III, Kolonne II, so erhält man die in Tabelle IV aufgeführten Cumarinverbindungen, welche ähnliche Eigenschaften aufweisen.

Tabelle IV

| Nr. | Cumarinverbindung | Nuance auf Polyester |
|---|---|---|
| 27 | 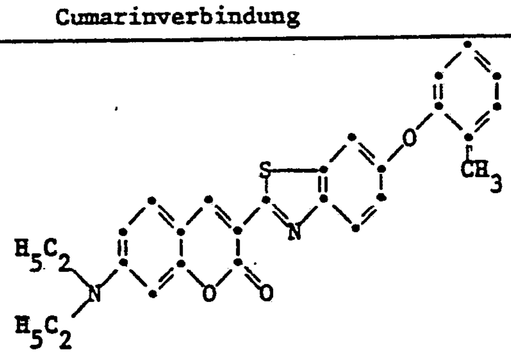 | grünstichig-gelb |
| 28 | 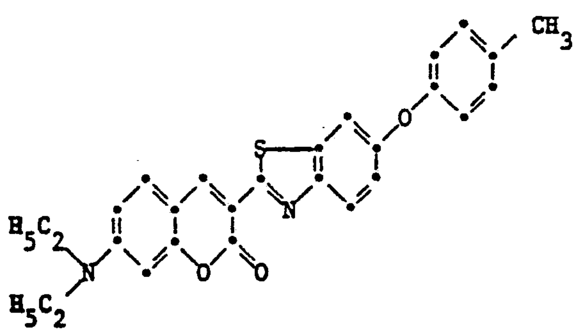 | do. |

Tabelle IV (Fortsetzung)

| Nr. | Cumarinverbindung | Nuance auf Polyester |
|---|---|---|
| 29 | 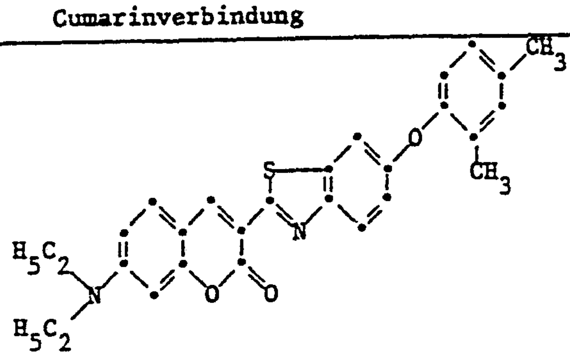 | grünstichig-gelb |
| 30 | 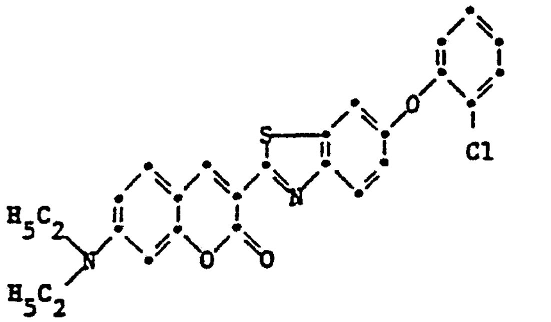 | do. |
| 31 | 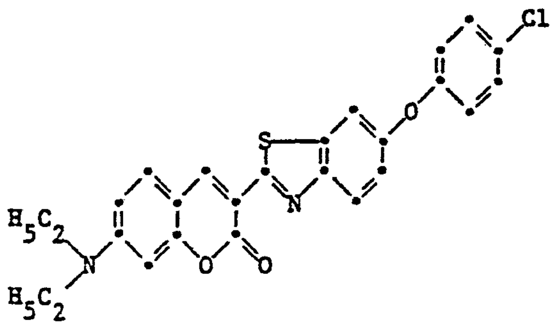 | do. |
| 32 | 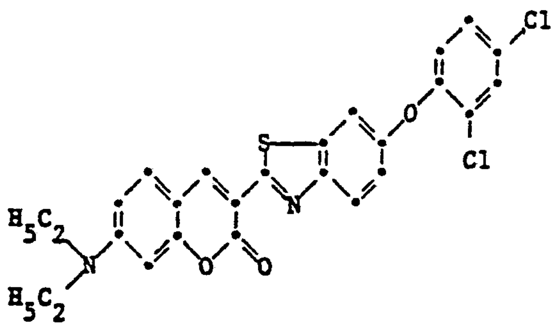 | do. |

Tabelle IV (Fortsetzung)

| Nr. | Cumarinverbindung | Nuance auf Polyester |
|---|---|---|
| 33 | 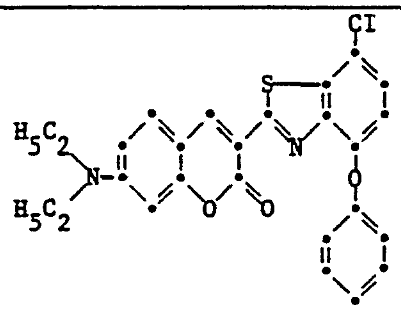 | grünstichig-gelb |
| 34 | 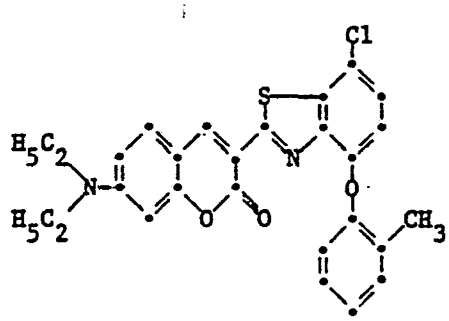 | do. |
| 35 | 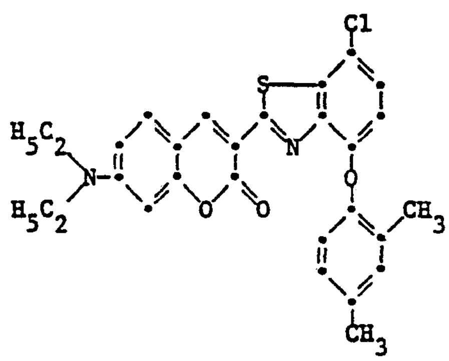 | do. |

Beispiel 36

Man trägt unter Rühren 4 g der Verbindung der Formel

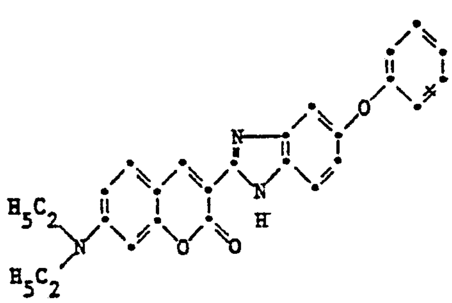

in 40 g Schwefelsäure-Monohydrat ein und rührt anschliessend das Gemisch noch während 6 Std. bei Raumtemperatur, worauf man dieses in eine Mischung aus 80 g Eis, 9 g Kochsalz und 10 ml Wasser

giesst. Anschliessend filtriert man die gebildete Fällung auf einer Nutsche ab und wäscht zweimal mit wenig 10 %iger Kochsalzlösung nach. Man saugt gut ab, schlämmt hierauf das Nutschgut in 50 ml Wasser an und stellt den pH-Wert des Gemisches durch Zugabe von verdünnter Natronlauge auf 7,0. Man filtriert hierauf das Natriumsalz der ausgefällten Cumarinverbindung der Formel

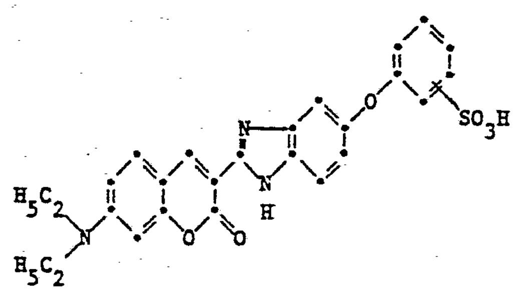

ab und trocknet dieses im Vakuum bei 70°. Das so erhaltene Natriumsalz der Cumarinverbindung bildet ein gelbes Pulver, welches sich in Wasser mit gelber Farbe löst.

Auf Polyamidgewebe aus schwach saurem Bad appliziert erhält man grünstichig-gelbe Färbungen mit intensiver Fluoreszenz, welche sich durch eine gute Lichtechtheit und gute Nassechtheiten auszeichnen. Der Farbstoff zeigt einen hohen Ausziehgrad und gutes Aufbauvermögen, und die Färbungen weisen eine gute Egalität auf.

Verwendet man, bei ansonst gleichem Vorgehen, anstelle der oben aufgeführten Verbindungen aequivalente Mengen der Cumarinverbindungen der Tabelle II, so erhält man die Cumarinverbindungen der Tabelle V, welche ähnliche Eigenschaften aufweisen.

Tabelle V

| Nr. | Cumarinverbindung | Nuance auf Polyamid |
|---|---|---|
| 37 | 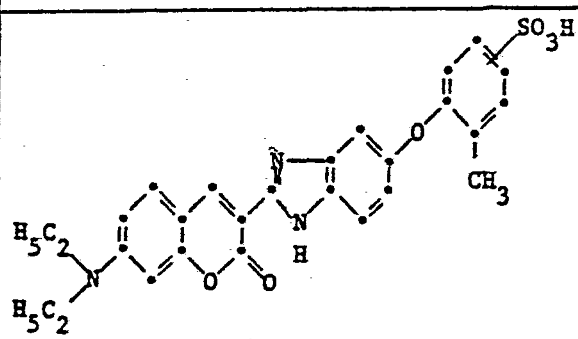 | grünstichig-gelb |
| 38 | 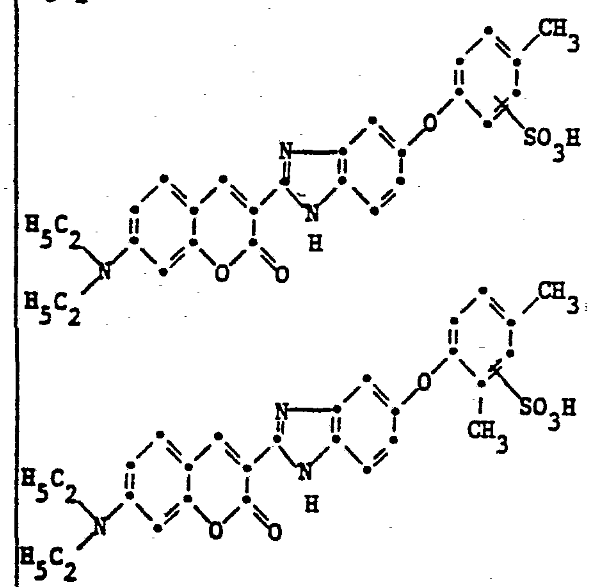 | do. |
| 39 | | do. |

Tabelle V (Fortsetzung)

| Nr. | Cumarinverbindung | Nuance auf Polyamid |
|---|---|---|
| 40 | 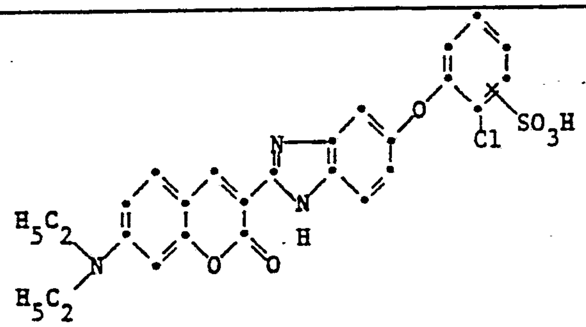 | grünstichig-gelb |
| 41 | 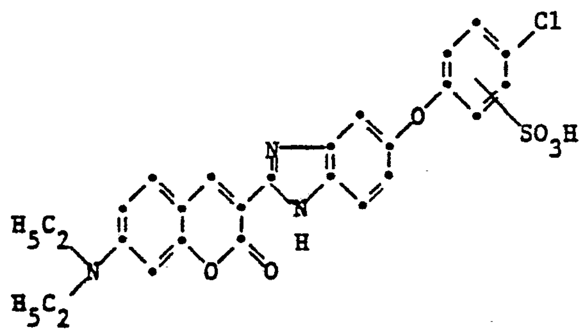 | do. |
| 42 | 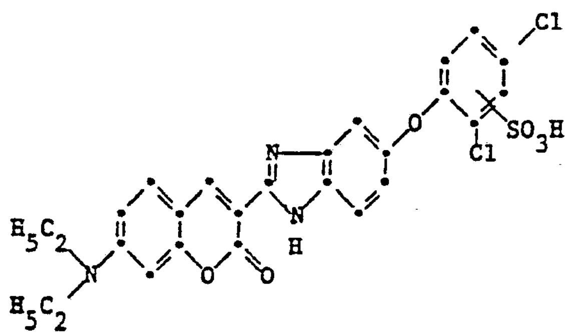 | do. |

Beispiel 43

Man trägt 4 g der Verbindung der Formel

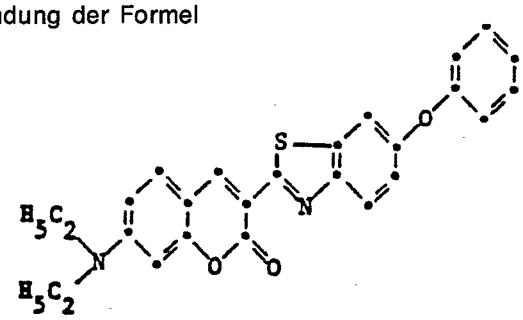

bei Raumtemperatur in 40 g Schwefelsäure-Monohydrat ein und rührt anschliessend das Gemisch noch während 6 Std. bei derselben Temperatur, worauf man es in eine Mischung bestehend aus 20 ml Wasser, 60 g Eis und 4 g Kochsalz giesst. Anschliessend filtriert man die gebildete Fällung auf einer Nutsche ab und wäscht mit 5 %iger Natriumchlorid-Lösung nach. Man saugt gut ab und schlämmt das Nutschgut hierauf in 100 ml Wasser an, worauf man das Gemisch mit verdünnter Natronlauge auf den pH-Wert 7,0 stellt. Man heizt auf 50° auf, wobei fast alles in Lösung geht, lässt dann unter Rühren erkalten und gibt bei Erreichen von 40° 7 g Kochsalz zu. Man filtriert die gebildete Fällung ab und trocknet das so erhaltene Natriumsalz der Cumarinverbindung der Formel

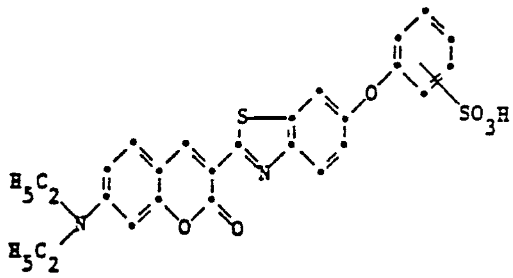

im Vakuum bei 70°.

Man erhält ein orange-farbiges Pulver, welches sich in Wasser grünstichig-gelb löst.

Aus schwach saurem Bad auf Polyamid-Gewebe appliziert, erhält man grünstichig-gelbe Färbungen mit intensiver Fluoreszenz, welche sich durch eine gute Lichtechtheit und gute Nassechtheiten auszeichnen. Der Farbstoff zeigt ein gutes Auszieh- und Aufbauvermögen, die erhaltenen Färbungen zeigen eine gute Egalität.

Verwendet man, bei sonst gleichem Vorgehen, anstelle der oben angeführten Verbindung aequivalente Mengen der Cumarin-Verbindungen der Tabelle IV, so erhält man die Cumarinverbindungen der Tabelle VI, welche ähnliche Eigenschaften aufweisen.

Tabelle VI

| Nr. | Cumarinverbindungen | Nuance auf Polyamid |
|---|---|---|
| 44 | 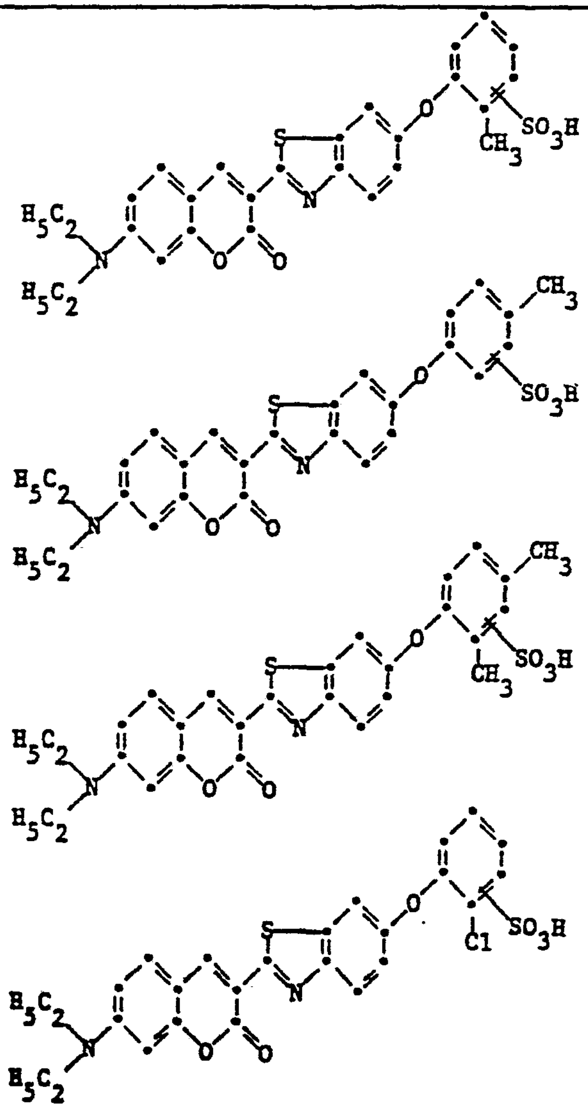 | grünstichig-gelb |
| 45 | | do. |
| 46 | | do. |
| 47 | | do. |

Tabelle VI (Fortsetzung)

| Nr. | Cumarinverbindung | Nuance auf Polyamid |
|---|---|---|
| 48 | 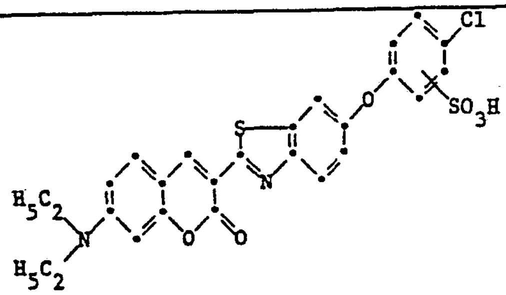 | grünstichig-gelb |
| 49 | 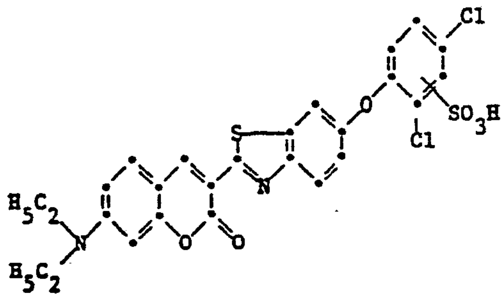 | do. |
| 50 | 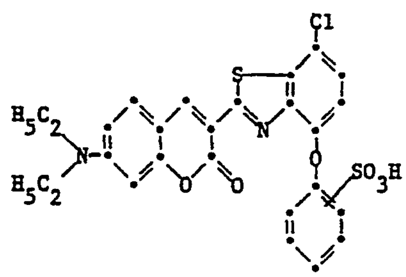 | do. |
| 51 | 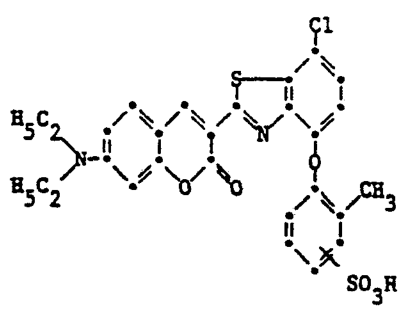 | do. |
| 52 | 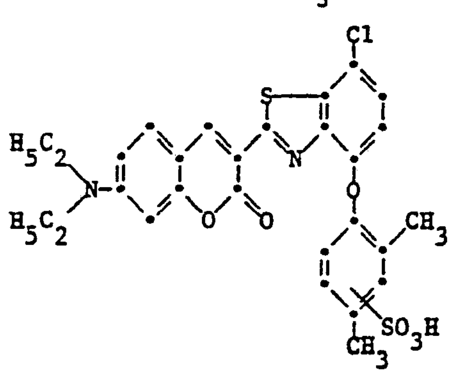 | grünstichg-gelb |

Beispiel 53

Man löst 13,3 g der Verbindung der Formel

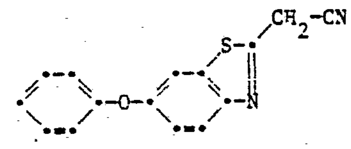

bei Raumtemperatur in 75 g Schwefelsäure-Monohydrat und rührt die erhaltene Lösung anschliessend während 6 Stunden bei einer Temperatur von 20-25°. Man lässt hierauf 50 ml Wasser so zutropfen, dass die Temperatur bis maximal 60° steigt.

Man trägt nun 10,25 g N-Diäthylaminosalicylaldehyd ein, worauf sich die Lösungsfarbe nach rot ändert. Man erhöht nun die Temperatur auf 80° und rührt die Lösung während 6 Std. bei 78-80°.

Man lässt auf Raumtemperatur erkalten und giesst die Lösung auf eine Mischung bestehend aus 40 g Eis und 130 ml Wasser. Man filtriert die gebildete rote Suspension auf einer Nutsche und presst gut ab.

Das Nutschgut schlämmt man in einem Becherglas mit 200 ml Wasser an und stellt den pH-Wert des Gemisches durch Zugabe von Natronlauge auf 7. Man erhitzt nun auf 65°, lässt bei langsamem Rühren erkalten und gibt bei 45° 5 Vol.% Kochsalz zu. Man lässt noch während 1 Std. rühren und saugt hierauf das ausgefällte Natriumsalz des Farbstoffs der Formel

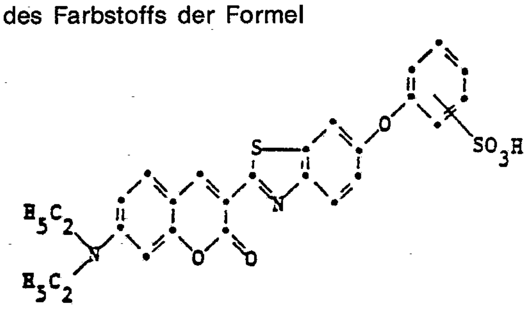

auf eine Nutsche ab und trocknet es anschliessend. Der so hergestellte Farbstoff ist mit dem nach Beispiel 43 erhaltenen identisch.

Beispiel 54

2 Teile der gemäss Beispiel 26 erhaltenen Cumarinverbindung werden in 4 000 Teilen Wasser dispergiert. Zu dieser Dispersion gibt man 12 Teile des Natriumsalzes von o-Phenylphenol sowie 12 Teile Diammoniumphosphat und färbt 100 Teile Garn aus Polyäthylenglykolterephthalat 90 Minuten lang bei 95 bis 98 °C in dieser Flotte.

Die Färbung wird anschliessend gut gespült und mit wässeriger Natronlauge und einem Dispergator nachbehandelt. Man erhält so eine fluoreszierende grünstichige gelbe Färbung.

Beispiel 55

1 Teil der gemäss Beispiel 26 erhaltenen Cumarinverbindung wird mit 2 Teilen einer 50 %igen wässerigen Lösung des Natriumsalzes der Dinaphthylmethandisulfonsäure nass vermahlen und getrocknet.

Dieses so erhaltene Präparat wird mit 40 Teilen einer 10 %igen wässrigen Lösung des Natriumsalzes der N-Benzylheptadecyl-benzimidazoldisulfonsäure verrührt und 4 Teile einer 40 %igen Essigsäurelösung zugegeben. Durch Verdünnen mit Wasser wird daraus ein Färbebad von 4 000 Teilen bereitet.

In dieses Bad geht man bei 50° mit 100 Teilen einer Polyesterfaser ein, steigert die Temperatur innert einer halben Stunde auf 120 bis 130° und färbt eine Stunde in geschlossenem Gefäss bei dieser Temperatur. Anschliessend wird gut gespült. Man erhält eine kräftige grünstichig gelbe Färbung.

Beispiel 56

Polyäthylenglykolterephthalatgewebe wird auf einem Foulard bei 40° mit einer Flotte folgender Zusammensetzung imprägniert:

20 Teile der gemäss Beispiel 26 erhaltenen Cumarinverbindung fein dispergiert in
10 Teilen Natriumalginat
20 Teilen Triäthanolamin
20 Teilen Octylphenolpolyglykoläther und
930 Teilen Wasser.

Das auf ca. 100 % abgequetschte Gewebe wird bei 100° getrocknet und anschliessend während 30 Sekunden bei einer Temperatur von 210° fixiert. Die gefärbte Ware wird mit Wasser gespült, geseift und getrocknet. Man erhält eine grünstichig gelbe Färbung.

Beispiel 57

Man bereitet ein Färbebad aus 4 000 Teilen Wasser, 4 Teilen Ammoniumacetat, 2 Teilen der sulfogruppenhaltigen Cumarinverbindung gemäss Beispiel 43 oder 53 und soviel Essigsäure, dass der pH-Wert des Bades 4,5 beträgt. In das erhaltene Färbebad geht man mit 100 Teilen eines synthetischen Polyamidtricots ein, erhitzt es innerhalb einer halben Stunde zum Kochen und färbt 45 Minuten bei 100°. Man erhält eine fluoreszierende grünstichig gelbe Färbung.

**Ansprüche**

1. Cumarinverbindungen der Formel I

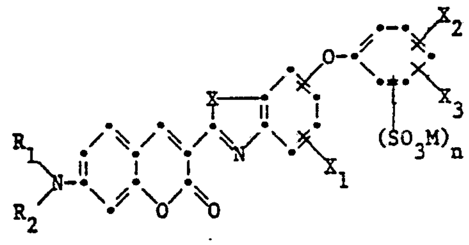

(I)

worin bedeuten :

$R_1$ und $R_2$ unabhängig voneinander einen Alkylrest mit 1 bis 4 Kohlenstoffatomen, X das —S-Atom oder die —NH-Gruppe, $X_1$ Wasserstoff oder Halogen, $X_2$ und $X_3$ unabhängig voneinander Wasserstoff, einen Alkylrest mit 1 bis 4 Kohlenstoffatomen oder Halogen, n entweder 0 oder 1, und M Wasserstoff oder ein Aequivalent eines Kations.

2. Cumarinverbindungen gemäss Anspruch 1, dadurch gekennzeichnet, dass $R_1$ und $R_2$ identisch sind.

3. Cumarinverbindungen gemäss Anspruch 2, dadurch gekennzeichnet, dass $R_1$ und $R_2$ je Aethyl bedeutet.

4. Cumarinverbindungen gemäss Anspruch 1, dadurch gekennzeichnet, dass $X_1$ Wasserstoff bedeutet.

5. Cumarinverbindungen gemäss Anspruch 1, dadurch gekennzeichnet, dass $X_2$ und $X_3$ je Wasserstoff bedeuten.

6. Cumarinverbindungen gemäss Anspruch 1, dadurch gekennzeichnet, dass n die Zahl 1 bedeutet.

7. Cumarinverbindung gemäss Anspruch 1, dadurch gekennzeichnet, dass diese der Formel

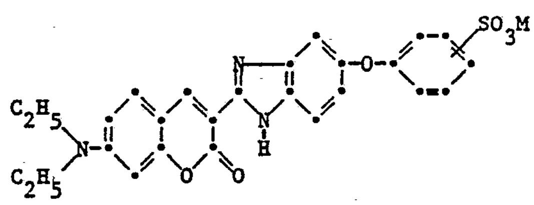

entspricht, worin M Wasserstoff oder ein Aequivalent eines Kations bedeutet.

8. Cumarinverbindung gemäss Anspruch 1, dadurch gekennzeichnet, dass diese der Formel

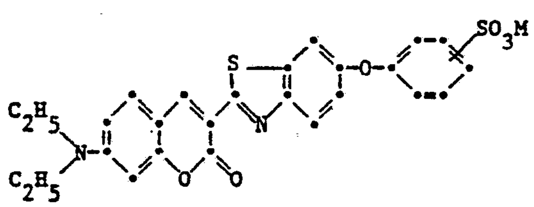

entspricht, worin M Wasserstoff oder ein Aequivalent eines Kations bedeutet.

9. Cumarinverbindung gemäss Anspruch 1, dadurch gekennzeichnet, dass diese der Formel

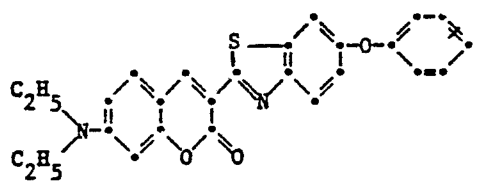

entspricht.

10. Cumarinverbindung gemäss Anspruch 1, dadurch gekennzeichnet, dass diese der Formel

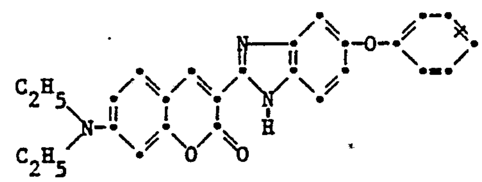

entspricht.

11. Verfahren zur Herstellung von Cumarinverbindungen der Formel I gemäss Anspruch 1, dadurch gekennzeichnet, dass man

a) heterocyclische Produkte der Formel II

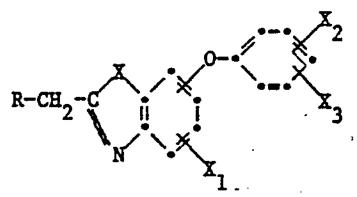

(II)

worin X, $X_1$, $X_2$ und $X_3$ die in Anspruch 1 angegebene Bedeutung haben und R Alkoxycarbonyl, Carbamoyl oder CN bedeutet mit einem Aldehyd der Formel III

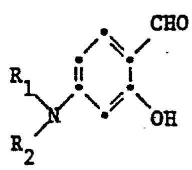

(III)

worin $R_1$ und $R_2$ die in Anspruch 1 angegebene Bedeutung haben, kondensiert und das Kondensationsprodukt gegebenenfalls sulfiert, oder

b) dass man eine heterocyclische Verbindung der Formel II sulfiert und nach der Sulfierung mit einem Aldehyd der Formel III kondensiert, wobei man Cumarinverbindungen der Formel I erhält worin n die Zahl 1 bedeutet.

12. Verfahren gemäss Anspruch 11, dadurch gekennzeichnet, dass man von einem heterocyclischen Produkt der Formel II ausgeht, worin $X_1$ Wasserstoff bedeutet.

13. Verfahren gemäss Anspruch 11, dadurch gekennzeichnet, dass man von einem heterocyclischen Produkt der Formel II ausgeht, worin $X_2$ und $X_3$ je Wasserstoff bedeuten.

14. Verfahren gemäss Anspruch 11, dadurch gekennzeichnet, dass man von einem Aldehyd der Formel III ausgeht worin $R_1$ und $R_2$ identisch sind.

15. Verfahren gemäss Anspruch 11, dadurch gekennzeichnet, dass man von einem Aldehyd der Formel III ausgeht, worin $R_1$ und $R_2$ je Aethyl bedeuten.

16. Verfahren gemäss Anspruch 11, dadurch gekennzeichnet, dass man Cumarinverbindungen der Formel I herstellt worin n die Zahl 1 bedeutet.

17. Verfahren gemäss Anspruch 11, dadurch gekennzeichnet, dass man Cumarinverbindungen der Formel

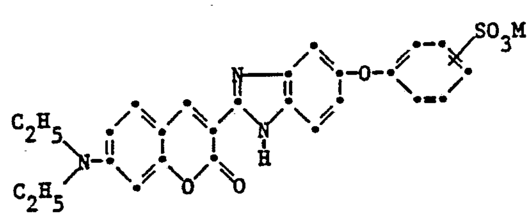

herstellt, worin M Wasserstoff oder ein Aequivalent eines Kations bedeutet.

18. Verfahren gemäss Anspruch 11, dadurch gekennzeichnet, dass man Cumarinverbindungen der Formel

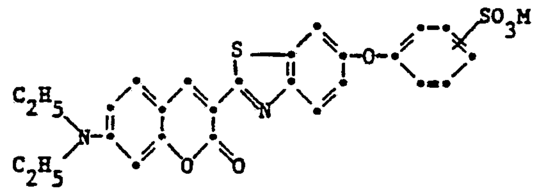

herstellt, worin M Wasserstoff oder ein Aequivalent eines Kations bedeutet.

19. Verfahren gemäss Anspruch 11, dadurch gekenzeichnet, dass man eine Cumarinverbindung der Formel

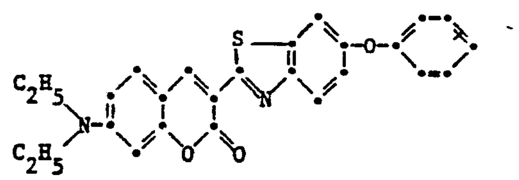

herstellt.

20. Verfahren gemäss Anspruch 11, dadurch gekennzeichnet, dass man eine Cumarinverbindung der Formel

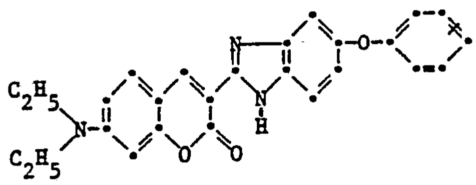

herstellt.

21. Verwendung der Cumarinverbindungen der Formel I gemäss Anspruch 1, worin n = 0 bedeutet als Dispersionsfarbstoffe zum Färben und Bedrucken von Materialien, insbesondere Textilmaterialien welche mit Dispersionsfarbstoffen färbbar sind.

22. Verwendung gemäss Anspruch 21, zum Färben und Bedrucken von Polyestermaterialien.

23. Verwendung der Cumarinverbindungen der Formel I gemäss Anspruch 1, worin n = 1 bedeutet als saure Farbstoffe zum Färben und Bedrucken von Materialien, insbesondere Textilmaterialien, welche mit sauren Farbstoffen färbbar sind.

24. Verwendung gemäss Anspruch 23 zum Färben und Bedrucken von Polyamidmaterialien oder Wolle.

25. Die mittels der Cumarinverbindungen gemäss Anspruch 1 gefärbten und bedruckten Materialien.

26. Zwischenprodukte der Formel

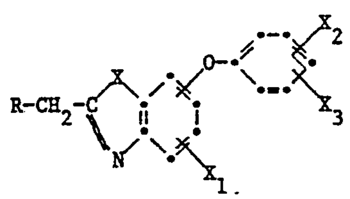

worin bedeuten :

R Alkoxycarbonyl, Carbamoyl oder CN, X das —S-Atom oder die —NH-Gruppe, $X_1$ Wasserstoff oder Halogen, und $X_2$ und $X_3$ unabhängig voneinander Wasserstoff, einen Alkylrest mit 1 bis 4 Kohlenstoffatomen oder Halogen.

**Claims**

1. A coumarin compound of the formula I

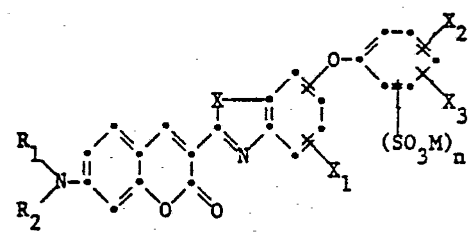

(I)

wherein

$R_1$ and $R_2$ independently of one another are each an alkyl group having 1 to 4 carbon atoms, X is the S atom or the NH group, $X_1$ is hydrogen or halogen, $X_2$ and $X_3$ independently of one another are each hydrogen, an alkyl group having 1 to 4 carbon atoms, or halogen, n is either nought or 1, and M is hydrogen or an equivalent of a cation.

2. A coumarin compound according to Claim 1, wherein $R_1$ and $R_2$ are identical.

3. A coumarin compound according to Claim 2, wherein $R_1$ and $R_2$ are each ethyl.

4. A coumarin compound according to Claim 1, wherein $X_1$ is hydrogen.

5. A coumarin compound according to Claim 1, wherein $X_2$ and $X_3$ are each hydrogen.

6. A coumarin compound according to Claim 1, wherein n is the number 1.

7. A coumarin compound according to Claim 1, which compound corresponds to the formula

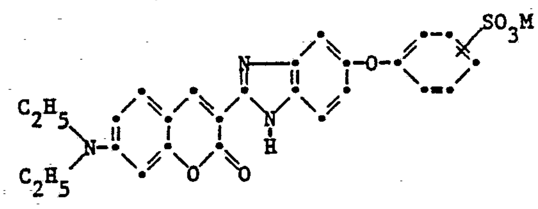

wherein M is hydrogen or an equivalent of a cation.

8. A coumarin compound according to Claim 1, which compound corresponds to the formula

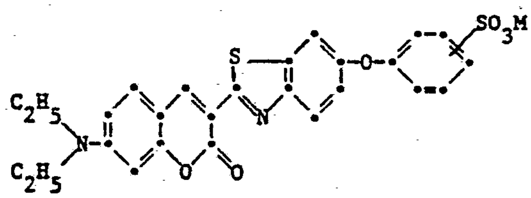

wherein M is hydrogen or an equivalent of a cation.

9. A coumarin compound according to Claim 1, which compound corresponds to the formula

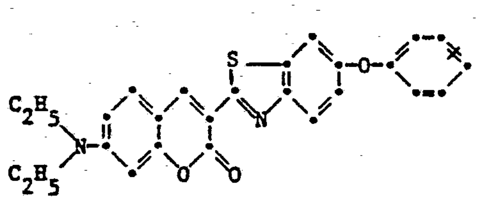

10. A coumarin compound according to Claim 1, which compound corresponds to the formula

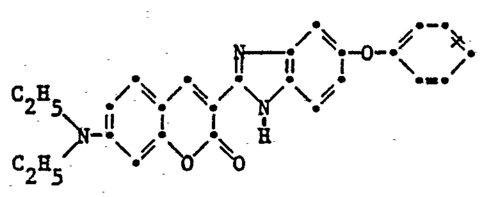

11. A process for producing a coumarin compound of the formula I according to Claim 1, which process comprises

a) condensing a heterocyclic compound of the formula II

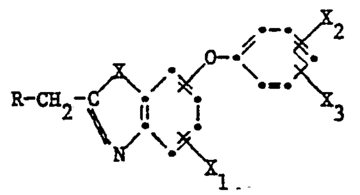

(II)

wherein X, $X_1$, $X_2$ and $X_3$ have the meanings given in Claim 1, and R is alkoxycarbonyl, carbamoyl or CN, with an aldehyde of the formula III

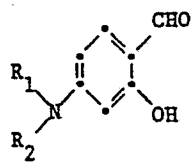

(III)

wherein $R_1$ and $R_2$ have the meanings given in Claim 1, and optionally sulfonating the condensation product ; or

b) sulfonating a heterocyclic compound of the formula II and, after sulfonation, condensing the sulfonation product with an aldehyde of the formula III, by which method there is obtained a coumarin compound of the formula I wherein n is the number 1.

12. A process according to Claim 11, wherein a starting material is a heterocyclic product of the formula II in which $X_1$ is hydrogen.

13. A process according to Claim 11, wherein a starting material is a heterocyclic product of the formula II in which $X_2$ and $X_3$ are each hydrogen.

14. A process according to Claim 11, wherein a starting material is an aldehyde of the formula III in which $R_1$ and $R_2$ are identical.

15. A process according to Claim 11, wherein a starting material is an aldehyde of the formula III in which $R_1$ and $R_2$ are each ethyl.

16. A process according to Claim 11, wherein there is produced a coumarin compound of the formula I in which n is the number 1.

17. A process according to Claim 11, wherein there is produced a coumarin compound of the formula

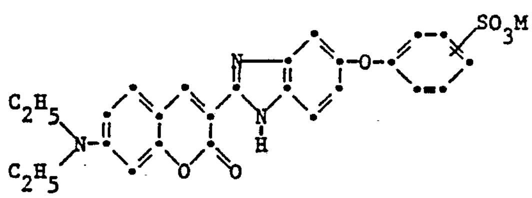

in which M is hydrogen or an equivalent of a cation.

18. A process according to Claim 11, wherein there is produced a coumarin compound of the formula

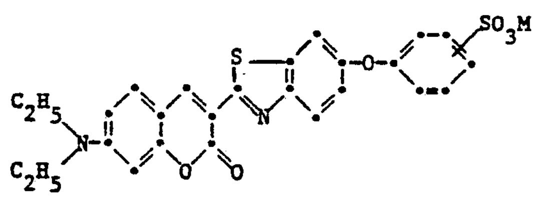

in which M is hydrogen or an equivalent of a cation.

19. A process according to Claim 11, wherein there is produced a coumarin compound of the formula

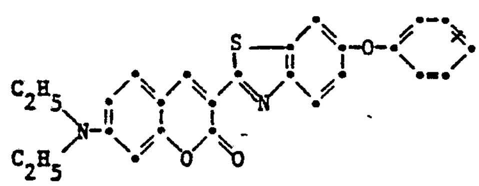

20. A process according to Claim 11, wherein there is produced a coumarin compound of the formula

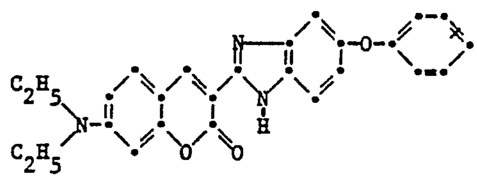

21. Use of a coumarin compound of the formula I according to Claim 1, in which n is nought, as a disperse dye for dyeing and printing materials, particularly textile materials, which are dyeable with disperse dyes.

22. Use according to Claim 21 for dyeing and printing polyester materials.

23. Use of a coumarin compound of the formula I according to Claim 1, in which n is 1, as an acid dye for dyeing and printing materials, particularly textile materials, which are dyeable with acid dyes.

24. Use according to Claim 23 for dyeing and printing polyamide materials or wool.

25. The materials dyed and printed by means of the coumarin compounds according to Claim 1.

26. An intermediate product of the formula

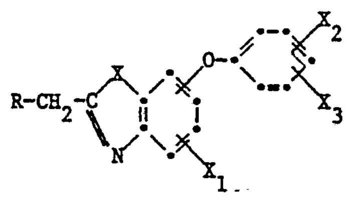

wherein

R is alkoxycarbonyl, carbamoyl or CN, X is the S atom or the NH group, $X_1$ is hydrogen or halogen, and $X_2$ and $X_3$ independently of one another are each hydrogen, an alkyl group having 1 to 4 carbon atoms, or halogen.

**Revendications**

1. Composés de coumarine de formule I

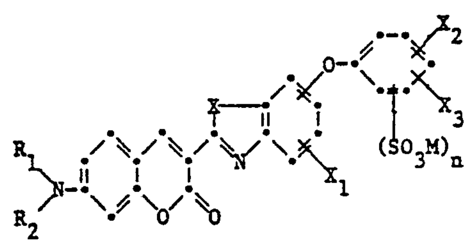

(I)

dans laquelle

$R_1$ et $R_2$ indépendamment l'un de l'autre sont des restes alkyle ayant 1 à 4 atomes de carbone ; X est l'atome —S ou le groupe —NH ; $X_1$ est de l'hydrogène ou de l'halogène ; $X_2$ et $X_3$ indépendamment l'un de l'autre sont de l'hydrogène, des restes alkyle ayant 1 à 4 atomes de carbone ou des atomes d'halogène ; n vaut 0 ou 1, et M est de l'hydrogène ou un équivalent d'un cation.

2. Composés de coumarine selon la revendication 1, caractérisés par le fait que $R_1$ et $R_2$ sont identiques.

3. Composés de coumarine selon la revendication 2, caractérisés par le fait que $R_1$ et $R_2$ sont chacun un groupe éthyle.

4. Composés de coumarine selon la revendication 1, caractérisés par le fait que $X_1$ est de l'hydrogène.

5. Composés de coumarine selon la revendication 1, caractérisés par le fait que $X_2$ et $X_3$ sont chacun de l'hydrogène.

6. Composés de coumarine selon la revendication 1, caractérisés par le fait que n est le nombre 1.

7. Composé de coumarine selon la revendication 1, caractérisé par le fait que celui-ci correspond à la formule

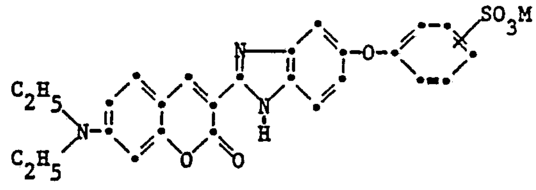

dans laquelle M est de l'hydrogène ou un équivalent d'un cation.

8. Composé de coumarine selon la revendication 1, caractérisé par le fait que celui-ci correspond à la formule

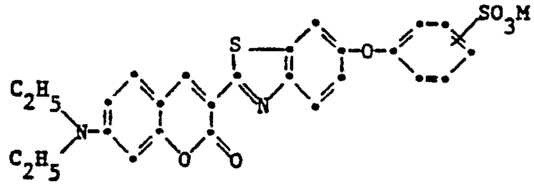

dans laquelle M est de l'hydrogène ou un équivalent d'un cation.

9. Composé de coumarine selon la revendication 1, caractérisé par le fait que celui-ci correspond à la formule

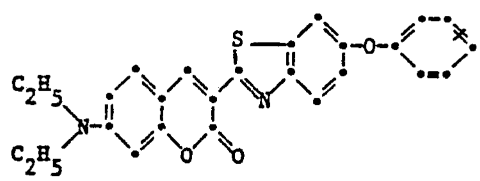

10. Composé de coumarine selon la revendication 1, caractérisé par le fait que celui-ci correspond à la formule

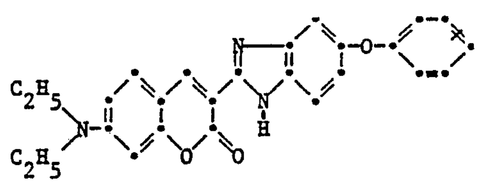

11. Procédé pour la préparation des composés de coumarine de formule I selon la revendication 1, caractérisé par le fait que

a) on condense des produits hétérocycliques de formule II

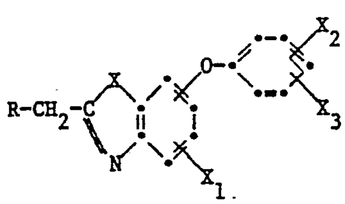

(II)

dans laquelle X, $X_1$, $X_2$ et $X_3$ ont la signification donnée dans la revendication 1, et R est un groupe alcoxycarbonyle, carbamoyle ou CN, avec un aldéhyde de formule III

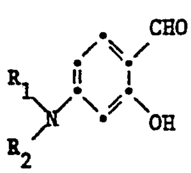

(III)

dans laquelle $R_1$ et $R_2$ ont les significations données dans la revendication 1, puis qu'on sulfone éventuellement le produit de condensation ou bien

b) qu'on sulfone un composé hétérocyclique de formule II et qu'après la sulfonation on condense avec un aldéhyde de formule III, ce qui fait qu'on obtient des composés de coumarine de formule I dans lesquels n désigne le nombre 1.

12. Procédé selon la revendication 11, caractérisé par le fait qu'on part d'un produit hétérocyclique de formule II, dans lequel $X_1$ est de l'hydrogène.

13. Procédé selon la revendication 11, caractérisé par le fait qu'on part d'un produit hétérocyclique de formule II, dans lequel $X_2$ et $X_3$ désignent chacun de l'hydrogène.

14. Procédé selon la revendication 11, caractérisé par le fait qu'on part d'un aldéhyde de formule III, dans lequel $R_1$ et $R_2$ sont identiques.

15. Procédé selon la revendication 11, caractérisé par le fait qu'on part d'un aldéhyde de formule III, dans lequel $R_1$ et $R_2$ sont chacun le groupe éthyle.

16. Procédé selon la revendication 11, caractérisé par le fait qu'on prépare des composés de coumarine de formule I dans lesquels n désigne le nombre 1.

17. Procédé selon la revendication 11, caractérisé par le fait qu'on prépare des composés de coumarine de formule

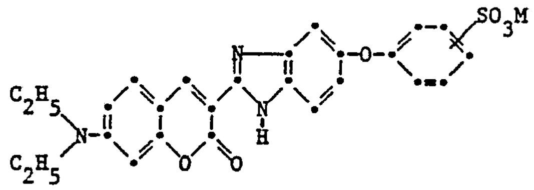

dans laquelle M est de l'hydrogène ou un équivalent d'un cation.

18. Procédé selon la revendication 11, caractérisé par le fait qu'on prépare des composés de coumarine de formule

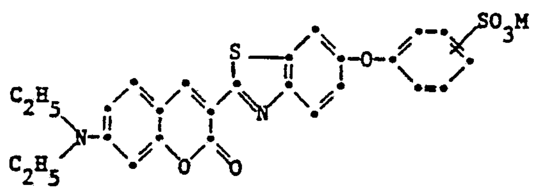

dans laquelle M est de l'hydrogène ou un équivalent d'un cation.

19. Procédé selon la revendication 11, caractérisé par le fait qu'on prépare un composé de coumarine de formule

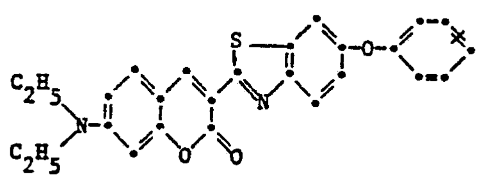

20. Procédé selon la revendication 11, caractérisé par le fait qu'on prépare un composé de coumarine de formule

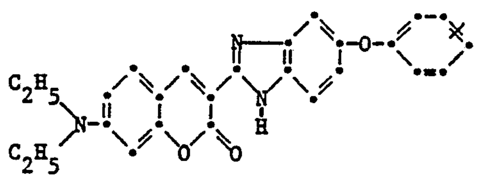

21. Utilisation des composés de coumarine de formule I selon la revendication 1, dans lesquels n désigne 0 comme colorants dispersés pour la teinture et l'impression de matières, en particulier de matières textiles qui peuvent être teintes avec des colorants dispersés.

22. Utilisation selon la revendication 21, pour la teinture et l'impression de matières en polyester.

23. Utilisation des composés de coumarine de formule I selon la revendication 1, dans lesquels n désigne 1 comme colorants acides pour la teinture et l'impression de matières, en particulier de matières textiles, qui peuvent être teintes avec des colorants acides.

24. Utilisation selon la revendication 23 pour la teinture et l'impression de matières en polyamide ou de la laine.

25. Les matières teintes et imprimées à l'aide des composés de coumarine selon la revendication 1.

26. Produits intermédiaires de formule

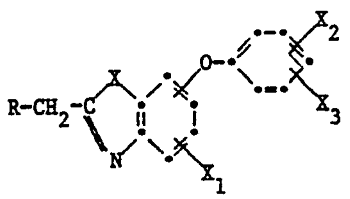

dans laquelle

R est un groupe alcoxycarbonyle, carbamoyle ou CN ; X est l'atome —S ou le groupe —NH ; $X_1$ est de l'hydrogène ou de l'halogène, et $X_2$ et $X_3$ indépendamment l'un de l'autre sont de l'hydrogène, des restes alkyle ayant 1 à 4 atomes de carbone ou des atomes d'halogène.